# EUROPEAN PATENT APPLICATION

(11) **EP 4 742 267 A1**
(43) Date of publication of application: **13.05.2026**
(21) Application number: 25214381.3
(22) Date of filing: 07.11.2025
(51) Int. Cl.: G16H 40/60, G16H 40/20, G16H 40/40

(54) **ALARM FATIGUE MITIGATION**

(30) Priority: 08.11.2024 US 202463717981 P
(71) Applicant: Drägerwerk AG & Co. KGaA, 23558 Lübeck (DE)
(72) Inventor: HARROD, John P., North Andover, 01845 (US)
(74) Representative: Haseltine Lake Kempner LLP

(57) **Abstract**

An Artificial Intelligence ("Al") solution that can be used to identify alarms that contribute to alarm fatigue is disclosed. Clinicians are directly involved in providing feedback that can be used to train supervised machine learnings models. These insights can then be used by the hospital to make data-driven actions for reducing the number of alarms generated in a hospital. The technological system described herein includes a machine learning ("ML") model deployed in a hospital that is provided with alarms and an associated feature set. The technological system embodies a method by which clinicians can flag potential nuisance alarms, thereby providing direct feedback to the system. The technological system also includes a recommender system that provides a prioritized set of potential nuisance alarms and recommendations.

## Description

### TECHNICAL FIELD

The present disclosure relates generally to the field of alarm management in a medical care context and, more particularly, to a method and apparatus for managing alarm fatigue.

### BACKGROUND

This section of this document introduces information about and/or from the art that may provide context for or be related to the subject matter described herein and/or claimed below. It provides background information to facilitate a better understanding of the various aspects of the present invention. This is a discussion of "related" art. That such art is related in no way implies that it is also "prior" art. The related art may or may not be prior art. The discussion in this section of this document is to be read in this light, and not as admissions of prior art.

Medical care environments can be full of alarms. Many pieces of equipment can issue a number of alarms for a variety of reasons. Many of these alarms may indicate a serious condition that needs to be addressed by a caregiver. Some of these alarms are "nuisance alarms". That is, they are either in error (a "false alarm") or indicate a condition that does not necessarily need to be addressed or, at least, not with much alacrity. The proliferation of alarms has led to a condition known as "alarm fatigue". Those in the art having the benefit of this disclosure will appreciate that whether any particular alarm is a "nuisance alarm" will depend to some degree on well-known factors such as the context in which an alarm occurs and on who is deciding whether the alarm is a nuisance. Some common characteristics of various kinds of nuisance alarms are set forth in Table 1 below.

**TABLE 1. Characteristics of Common Kinds of Nuisance Alarms**

| Characteristic | Description | Examples |
|---|---|---|
| False Alarms | Alarms triggered without a valid event, often due to technical issues. | Poor electrode contact, signal artifacts. |
| Nonactionable Alarms | True alarms that do not require clinical intervention. | Alarms triggered by minor fluctuations in heart rate that are not clinically significant. |
| High Frequency | Alarms that occur more frequently. | Frequent alarms due to overly sensitive settings. |
| Lack of Clinical Relevance | Alarms that do not provide useful information for patient care. | Alarms for minor, non-threatening changes in patient vitals. |

Alarm fatigue is a complex and pervasive problem that occurs when clinicians are exposed to excessive numbers of alarms, which can result in the desensitization to alarm sounds and an increased rate of missed alarms. This can lead adverse consequences. Research indicates that 72% to 99% of all alarms are false, which has led to the prevalence of alarm fatigue. Alarm fatigue is a sensory overload that occurs when clinicians are exposed to an excessive number of alarms, which can result in desensitization to alarm sounds and an increased rate of missed alarms.

The art therefore seeks to mitigate the occurrence and consequences of alarm fatigue. Some approaches attempt to mitigate some kinds of causes of nuisance alarms. For example, some approaches monitor and clean medical equipment as this kind of maintenance can help reduce the frequency of alerts related to technical malfunctions such as low battery or loose a connection. Some approaches seek to decrease clinically inconsequential alerts. One example includes reducing the number of alarms that are announced due to clinically inconsequential events. Still other approaches try to reduce false alarms since reducing the number of false alarms can help reduce alarm fatigue.

### SUMMARY

This disclosure describes an Artificial Intelligence ("Al") solution that can be used to identify alarms that contribute to alarm fatigue. Clinicians are directly involved in providing feedback that can be used to train supervised machine learnings models. These insights can then be used by the hospital to make data-driven actions for reducing the number of alarms generated in a hospital. The technological system described herein includes a machine learning ("ML") model deployed in a hospital that is provided with alarms and an associated feature set. The technological system embodies a method by which clinicians can flag potential nuisance alarms, thereby providing direct feedback to the system. The technological system also includes a recommender system that provides a prioritized set of potential nuisance alarms and recommendations.

Accordingly, a ML model is deployed in a hospital or other medical care facility and is provided with alarms and an associated feature set. Clinicians can flag potential nuisance alarms, thereby providing direct feedback to the system. A recommender system that provides a prioritized set of potential nuisance alarms and recommendations may also be provided.

In a first aspect, a computer-implemented method for managing alarm fatigue comprises: generating a set of candidate nuisance alarms from an aggregation of alarm data to recommend for clinician evaluation; receiving clinician evaluations for the recommended candidate nuisance alarms, the evaluations identifying the recommended candidate nuisance alarms as actual nuisance alarms or non-nuisance alarms; aggregating the identified nuisance and identified non-nuisance alarms into a cumulative set of alarm data; and iterating the above steps.

In second aspect, a method for use in reducing alarm fatigue comprises: separating a set of aggregated alarm data into a training subset and a testing subset; generating a set of candidate nuisance alarms by applying a machine learning model trained on the training subset to the testing subset to classify a plurality of alarms as nuisance alarms or non-nuisance alarms; and prioritizing the set of candidate nuisance alarms.

In third aspect, a method for use in reducing alarm fatigue comprises: presenting a prioritized set of candidate nuisance alarms for clinician evaluation, the set generated from identifying the candidate nuisance alarms in a set of aggregated alarm data; receiving clinician evaluations for the recommended candidate nuisance alarms, the evaluations identifying the recommended candidate nuisance alarms as actual nuisance alarms or non-nuisance alarms; and aggregating the identified nuisance and identified non-nuisance alarms into the set of aggregated alarm data.

In a fourth aspect, a computing system comprises one or more processors and a memory. On the memory resides an aggregation of alarm data and a set of instructions. The instructions, when executed by the one or more processors, performs a method for managing alarm fatigue comprises: generating a set of candidate nuisance alarms from the aggregation of alarm data to recommend for clinician evaluation; receiving clinician evaluations for the recommended candidate nuisance alarms, the evaluations identifying the recommended candidate nuisance alarms as actual nuisance alarms or non-nuisance alarms; aggregating the identified nuisance and identified non-nuisance alarms into a cumulative set of alarm data; and iterating the above steps.

In fifth aspect, a technological system comprises a plurality of medical devices and a computing apparatus. The plurality of medical devices are programmed to detect alarm conditions, announce alarms responsive to detecting alarm conditions, and transmit alarm data associated with the alarm. The computing apparatus comprises one or more processors and a memory. On the memory resides an aggregation of alarm data; and a set of instructions. The set of instruction, when executed by the one or more processors, performs a method for managing alarm fatigue. The method comprises: generating a set of candidate nuisance alarms from the aggregation of alarm data to recommend for clinician evaluation; receiving clinician evaluations for the recommended candidate nuisance alarms, the evaluations identifying the recommended candidate nuisance alarms as actual nuisance alarms or non-nuisance alarms; aggregating the identified nuisance and identified non-nuisance alarms into a cumulative set of alarm data; and iterating the above steps.

In a sixth aspect, a computer-implemented method for managing alarm fatigue in a medical monitoring environment comprises identifying nuisance alarms occurring in the medical monitoring environment and updating the programming of the plurality of medical devices to reflect the newly identified actual nuisance alarms. Identifying the nuisance alarms occurring in the medical monitoring environment, includes: harvesting alarm data from a plurality of medical devices; aggregating the alarm data; applying a trained machine learning model to the aggregated alarm data to generate a prioritized set of potential nuisance alarms; and receiving clinician feedback identifying actual nuisance alarms from among the potential nuisance alarms; and updating the programming of the plurality of medical devices to reflect the newly identified actual nuisance alarms.

A seventh aspect comprises a system implementing the method of the sixth aspect.

Further aspects comprise the computer-implemented method for managing alarm fatigue according to the appended independent claim 1, the computing system according to the appended independent claim 6, and the technological system according to the appended independent claim 12. Further optional features of each of these further aspects are described in the appended dependent claims 2 to 5, 7 to 11, and 13 to 15.

The above presents a simplified summary in order to provide a basic understanding of some aspects of what is claimed below. This summary is not an exhaustive overview of the claimed subject matter. It is not intended to identify key or critical elements of the disclosure or to delineate the scope of the claims. Its sole purpose is to present some concepts in a simplified form as a prelude to the more detailed description that is discussed below.

### BRIEF DESCRIPTION OF THE DRAWINGS

The detailed description is made with reference to the accompanying drawings and is provided to assist in a comprehensive understanding of various example embodiments of the present disclosure. Changes may be made in the function and arrangement of elements discussed without departing from the scope of the disclosure. Various embodiments may omit, substitute, or add various procedures or components as appropriate. For instance, features described with respect to certain embodiments may be combined in other embodiments. In addition, descriptions of well-known functions and constructions may be omitted for clarity and conciseness. Accordingly, those of ordinary skill in the art will recognize that various changes and modifications of the examples described herein can be made without departing from the scope of the present disclosure.

In the drawings, like reference numbers generally indicate identical, functionally similar, and/or structurally similar elements.
FIG. 1 conceptually illustrates a technological system according to one or more examples.
FIG. 2 depicts a scenario in which the technological system of FIG. 1 may be implemented according to one or more examples.
FIG. 3A, FIG. 3B, and FIG. 3C are block diagrams of selected aspects of the medical device, the server, and the workstation, respectively, first shown in FIG. 2.
FIG. 4 depicts a workflow as may be implemented in the scenario of FIG. 2 in some embodiments.
FIG. 5 depicts a workflow for training and deploying a machine learning model in accordance with some embodiments.
FIG. 6 depicts a scenario in which the technological system of FIG. 1 may be implemented according to one or more examples.

While the disclosed subject matter is susceptible to various modifications and alternative forms, the drawings illustrate specific implementations described in detail by way of example. It should be understood, however, that the description herein of specific examples is not intended to limit that which is claimed to the particular forms disclosed, but on the contrary, the intention is to cover all modifications, equivalents, and alternatives falling within the scope of the appended claims.

### DETAILED DESCRIPTION

Al can be utilized to help hospitals use these approaches to reduce the risk of alarm fatigue in the manner disclosed herein. With Al solutions/models, the quality is dependent on the data used to train it. Supervised machine learning ("ML") models are a popular technique used to build Al solutions. In general, machine learning involves training algorithms on labeled data to recognize patterns and predict outcomes.

More particularly, the typical development steps for supervised ML models are data collection, data cleaning, data splitting, model selection, model training, model evaluation, and model deployment. Data collection is what it sounds like-gathering labeled data, which typically consists of input features and their corresponding target labels. Data cleaning includes cleaning and organizing the collected data to ensure its quality and reliability. Data splitting divides the collected data into two subsets that will be called the training dataset and the testing dataset.

Once the data is split, model selection chooses an appropriate model. Options include decision trees, support vector machines and neural networks. The selected model is then trained-model training-on the training dataset. During training, the model learns to make predictions by adjusting its internal parameters. Model evaluation then evaluates the model using the test dataset to measure its performance. The evaluation metrics used depend on the problem being solved and the type of model being used. The model is then deployed in model deployment so that the model is then used in a real-world setting to make predictions on new data.

Once a supervised learning model is trained and deployed using the above steps, it may be used to identify nuisance alarms in hospital. When trained periodically with new data, the trained Al model could provide new and custom results. The insights generated by the trained Al model described in this disclosure may then be used as a stand-alone or be fed into a recommender system and/or existing solutions.

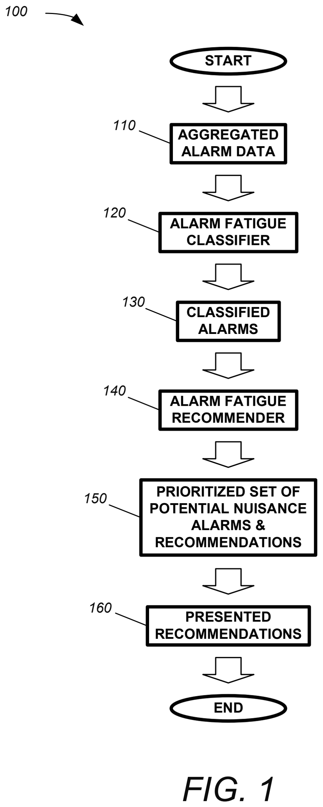

FIG. 1 conceptually illustrates a technological system according to one or more examples. The technological system 100 begins with the aggregation of alarm data (at 110). During the normal operation of a patient medical care system, not separately shown in FIG. 1, alarms are generated. The alarms may have varying degrees of significance. Some of these alarms may be minor, e.g., an intravenous ("IV") drip needs to be replenished, or a battery is getting low on charge and needs to be replaced or recharged. Some of these alarms may be major, e.g., the patient is in cardiac arrest or has stopped breathing.

Data regarding each of these alarms is aggregated in some repository, also not separately shown in FIG. 1. The identity and volume of alarm data aggregated for any particular alarm may vary by implementation, by alarm type, by priority, by type of device issuing the alarm, *etc.* The aggregated alarm data may also include any relevant conditions that triggered the alarm, such as a parameter value for a limit alert or a battery level for a low battery alert. Other data that may be accumulated may include the time of day the alarm triggered, the time elapsed since the last alarm of the same type was announced, time elapsed in which the alarm was active, and correlation with other active alarms, for example.

In some embodiments, the aggregated alarm data may also include contextual information for the alarm. For example, the location of the alarm may be aggregated. Other contextual information such as equipment configuration, equipment changes, configuration changes, maintenance, adjustments responsive to the alarm, and whether the alarm was flagged as a potential nuisance alarm. These are non-limiting examples of the kinds of alarm data that may be aggregated. Those in the art having the benefit of this disclosure will appreciate other kinds of alarm data that may be aggregated in addition to or in lieu of those set forth above. Furthermore, the amount and kinds of alarm data that are available may be limited by any number factors such as bandwidth, equipment capabilities, *etc.*

Although not shown in FIG. 1, at least a portion of the aggregated alarm data may also be used to train a ML model that will be used to classify alarms as discussed below. The aggregated alarm data may be separated into a training subset and a testing subset, the training subset labeled, and the ML model trained. The ML model may be tested and, once the ML model performs satisfactorily, the ML model is considered trained and is deployed.

A trained ML model, or "alarm fatigue classifier" 120, then classifies the alarms whose data is aggregated using (at 130) the alarm data previously aggregated as discussed above. Generally, the alarm fatigue classifier is looking for alarms that may be potential nuisance alarms and, so, the alarms may be classified as, for example, "potential nuisance alarms" "non-nuisance alarms". Some embodiments may use a larger number of classifications into smaller groupings with finer delineations amongst the groupings.

An alarm fatigue recommender 140 then operates on the classified alarms to develop a prioritized set of potential nuisance alarms and recommendations 150. The alarm fatigue recommender 140 may contain a rules-based engine that maps nuisance alarms to predetermined rules that guide the clinicians on how to eliminate or reduce the frequency of the alarm. After sorting suspected nuisance alarms by preconfigured criteria (count, priority, *etc*.), the rules could be presented to clinicians. Example rules are listed in Table 2 below. In some embodiments, alarms with no recommended course of action could be feedback back to Draeger for investigation resulting in rules updates via a service channel.

**Table 2. Examples Rules and Recommendations**

| Alarm | Recommended Actions |
|---|---|
| Low Battery Technical Alarm | Proactively replace battery on shift change. |
| | Check if battery is beyond expected lifetime and replace if needed. |
| Parameter High Limit | Adjust limit value by X% |
| Parameter Low Limit | |
| Hardware Failure | Replace Hardware (i.e. SpO2 pod) |

The potential nuisance alarms may be prioritized in a number of ways. For example, the trained ML model may prioritize based on the frequency and the confidence level that the model thinks an alarm is a nuisance. Or the potential nuisance alarms for which the system receives clinician feedback at the time the alarm occurs may receive a higher prioritization. The set of potential nuisance alarms may also be prioritized based on alarm severity (low/med/high), location and alarm type. Or priorities may be predicated on the severity of the potential impact on the patient's physical condition. Some embodiments may weigh these and, perhaps, still other considerations in sorting the potential nuisance alarms for presentation to the clinician.

The prioritized set of nuisance alarms and recommendations 150 is then presented to one or more clinicians (at 160) for input/feedback. More particularly, the prioritized set is a set of potential nuisance alarms and associated recommendations for confirmation or assessment by one or more clinicians. The clinician(s) can flag alarms as nuisance alarms and/or as potential nuisance alarms. The clinicians can also modify and/or propose recommendations for handling the alarms that are flagged or are presented but unflagged.

The clinician's input may be used in several ways. The clinician's input can be incorporated into the training set for the ML models to be used in training (or retraining) the alarm fatigue classifier. The clinician's input may also be used in programming various medical devices to recognize when an alarm is a nuisance alarm and how to respond. For example, a medical device might silence a nuisance alarm or silence a nuisance alarm and flag it for later attention and the clinician's input may be used to program this response. In the illustrated embodiments, the clinician input is used as input features to the ML model when the clinician silences (and potentially flags) the alarm as a nuisance and as labels for training after the prioritized list is reviewed.

The collection of data for inputting into the classifier could be done in the presently disclosed embodiments with an existing component such as a dedicated server, for example. Generally, to minimize any impact to patient monitoring, the Al models should not run on any computers that are used for clinical purposes and should be on a dedicated server or in a cloud. This dedicated server could also be the data aggregator. During the analysis of the recommender system's results, clinicians could label the new data which in turn could be used to retrain the classifier. As changes are made to address alarm fatigue, this would allow the classifier to adapt and gain new insights. Confirming and labeling nuisance alarms as part of the workflow to analyze existing results could minimize the amount of work as the clinicians would have prioritized sets of potential nuisance alerts that when confirmed, could be labeled using a batch process.

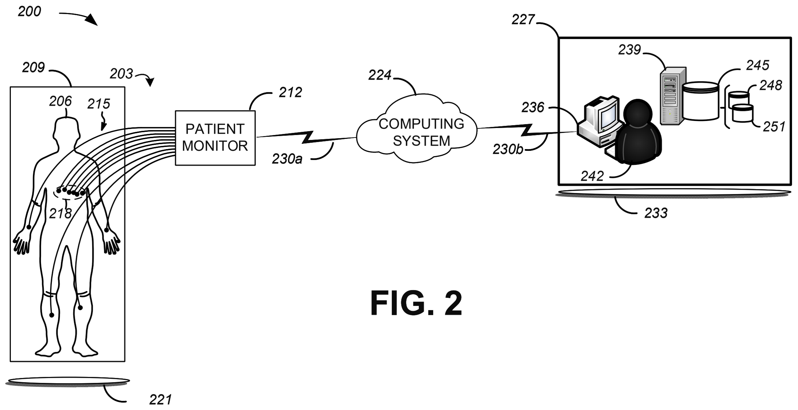

FIG. 2 depicts a scenario in which the technological system of FIG. 1 may be implemented according to one or more examples. In the scenario 200, the technological system includes a medical monitoring system 203. A patient 206 in a bed 209 physically interfaces with a medical device 212. Those in the art will appreciate that care may be delivered to a patient in a typical healthcare environment using a multiplicity of devices that might generate alarms. These alarms may be associated with the purpose and operation of the respective medical device and, so, may differ from those alarms issued by other devices.

Although not shown, those ordinarily skilled in the art having the benefit of this disclosure will appreciate that the medical monitoring system 203 will typically include a wide variety and number of other medical and network devices. Such other medical devices may include, for example and without limitation, IV drips, therapy devices, isolettes, movement detectors, etc. Other network devices may include, without limitation, gateways, antennas, servers, routers, and various other computing devices. These other medical and network devices work together and individually to impart functionality of the medical monitoring system 203 as described herein.

Accordingly, the medical device 212 in the scenario 200 is representative of a suite of medical devices that might be encountered in a typical healthcare environment. All behaviors and functionalities relevant to the claimed subject matter can be extrapolated to other medical devices that might be present but not shown. It is nevertheless to be understood that the issuance of alarms and the types of those alarms may vary depending on the medical device that issues the alarm.

The medical device 212 in the scenario 200 is a patient monitor monitoring one or more physiological parameters of the patient 206 but may be some other type of medical device in other embodiments. The physiological parameters are monitored by the medical device 212 over a plurality of leads 215 including sensors 218. The conductive leads 215 are affixed to the patient 206 disposed upon a bed 209 and data is being acquired. The patient 206 is located at what may be referred to as a "local" location 221, which is "local" because it is where the patient 206 is located.

Those in the art having the benefit of this disclosure will appreciate that a typical healthcare environment might monitor for a variety of physiological parameters such a heart rate, respiration rate, blood oxygen content, etc. In general terms, a patient monitor acquires data, processes and/or analyzes such acquired data. The processing and analysis might result in the detection of an alarm condition and, perhaps, the issuance of an alarm.

The scenario 200 includes not only the medical device 212 but also a computing system 224 and a work space 227. The work space 227 and the medical device 212 communicate with one another over the computing system 224, which includes the communications links 230a-230b. The work space 227 is at a remote location 233 relative to the local location 221. As used herein, in one sense, the term "remote" means a physically different location than where the medical device 212 is located. In one sense, "remote" may mean that the geographical location is physically different from the "local" location. In a second sense, "remote" also means that the location is outside the physical presence of the patient 206.

For example, patient 206 may be located in a room in which medical care is being delivered. The room may be in a medical care facility such as hospital, a hospice, an urgent care center, or a doctor's office, for example, depending on the implementation. The remote location may be in a different part of a municipality, or even in a different municipality or country than is the local location 221. The remote location 233 may be across town or down the street from the medial care facility in which the patient 206 is located. Or the "remote" location 233 may be in an office in the same building that the patient is being treated or down the hall from the room in which the patient 206 is located (i.e., the local location 221).

The computing system 224 may therefore be a combination of private and public networks. For example, the medical device 212 may communicate with the work space 227 over a medical facility's private network or over the Internet, which is a public network. Thus, communications between the medical device 212 and the work space 227 may occur in part or as a whole over private and/or public networks of the computing system 224. Accordingly, the communications links 230a-230b may be wired or wireless, depending on which is appropriate and/or desirable.

Alarm data from the medical monitoring system 203 is aggregated as discussed above. In this embodiment, the medical device 212 transmits the alarm data to the work space 227 over the computing system 212. The work space 227 includes at least two computing two computing apparatuses, a workstation 236 and a server 239. The server 239 hosts certain computing aspects of the technological system 100, shown in FIG. 1, and the clinician 242 interfaces with the software and aggregated data through the workstation 236. The server 239 aggregates the alarm data 245 in a suitable data structure.

In the interest of completeness, selected aspects of the medical device 212, the workstation 236, and the server 239 will now be presented. In general, it is contemplated by the present disclosure that medical device 212, the workstation 236, and the server 239, as well as any other computing device employed in the scenario 200, includes electronic components, software, and/or electronic computing devices operable to receive, transmit, process, store, and/or manage data and information associated performing the functions of the system as described herein. This contemplation encompasses any suitable processing device adapted to perform computing tasks consistent with the execution of computer-readable instructions stored in a memory or a computer-readable recording medium.

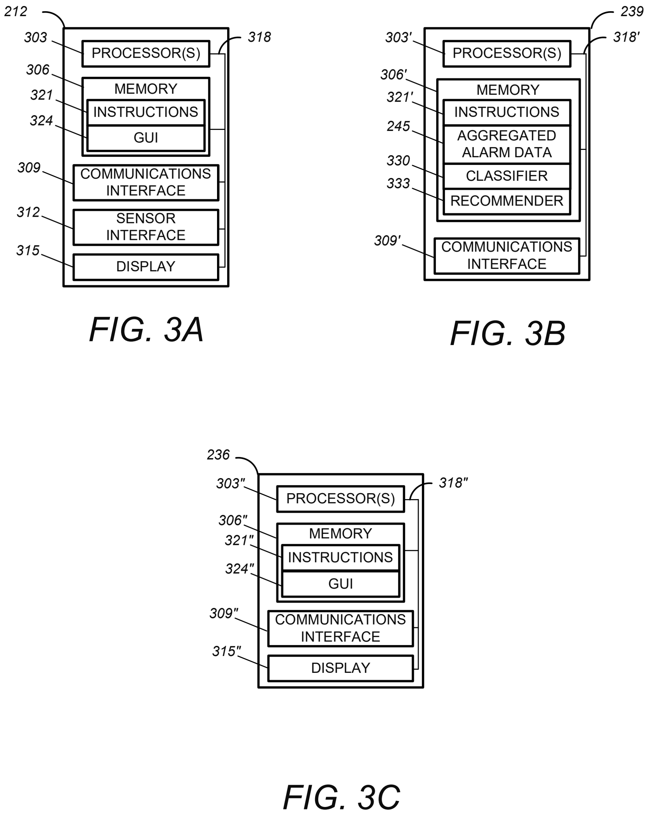

Referring now to FIG. 3A, the medical device 212 includes one or more processors 303, a memory 306, a communications interface 309, a sensor interface 312, a display 315, all communicating over a bus system 318. A set of instructions 321 and a graphical user interface ("GUI") 324 reside on the memory 306. The medical device 212 receives the data acquired by the sensors 218 and leads 215, both shown in FIG. 2, through the sensor interface 312. The processors 303 execute the instructions 321 to process and analyze the acquired data in real time or near real time. The processors 303 may then display the processed data to a caregiver (not shown) at the patient's bedside on the display 315 using the GUI 324. The processors 303 also, through the execution of the instructions 321, transmit the processed data off device using the communications interface. The alarm is then handled as described further below.

FIG. 3B shows the server 239, which includes one or more processors 303', a memory 306', and a communications interface 309' all communicating over a bus system 318'. A set of instructions 321' and a set of aggregated alarm data 245 reside on the memory 306'. The aggregated alarm data 245 may be stored in any suitable data structure known to the art. Also residing on the memory are the alarm fatigue classifier 330, or "classifier", and the alarm fatigue recommender 333, or "recommender". Those in the art will appreciate that servers are relatively large capacity computing devices and so the server 239 may include many more computational resources than are shown in addition to those that are shown.

FIG. 3C depicts the workstation 236. The workstation 236 includes one or more processors 303", a memory 306", a communications interface 309", and a display 315", all of which communicate with one another over a bus system 318". A set of instructions 321" and a GUI 324" reside on the memory 306". Execution of the instructions 321" by the processor(s) 318" imparts the functionality of the workstation 236, including interaction with the clinician 242, shown in FIG. 2, through the GUI 324". The clinician 242 may also invoke the classifier 330 and interact with the recommender 333, both shown in FIG. 3B, through the GUI 324".

Those in the art having the benefit of this disclosure will appreciate that the patient monitor 212, workstation 236, and server 239 may, and probably will, include other components. These other components may implement common functionalities, like a power source. For instance, a power source (not shown) may include a self-contained power source such as a battery pack and/or include an interface to be powered through an electrical outlet, either directly or by way of a monitor mount. The power source may also be a rechargeable battery that can be detached allowing for replacement. In the case of a rechargeable battery, a small built-in back-up battery (or super capacitor) can be provided for continuous power to be provided during battery replacement.

Some of these other components may differentiate medical and other computing devices for their intended functions. For example, some medical devices, such as patient monitors including sensor interfaces as described above through which they may receive sensed data. Other medical devices and computing devices may omit such sensor interfaces. For another example, some network devices may be part of a cloud computing system and therefore host virtualized components of a virtual machine. Those in the art having the benefit of this disclosure will appreciate still other examples of functionality differentiation among medical and network devices and concomitant the differentiation among components.

Returning now to FIG. 2 and FIG. 3A - FIG. 3C collectively, the one or more processors 303, 303, 303" may be used for controlling the general operations of the respective device 212, 236, 239. The one or more processors 303, 303', 303" may be any suitable processor-based resource. They may be, but are not limited to, a central processing unit ("CPU"), a hardware microprocessor, a multi-core processor, a single core processor, a field programmable gate array ("FPGA"), a controller, a microcontroller, an application specific integrated circuit ("ASIC"), a digital signal processor ("DSP"), or other similar processing device capable of executing any type of instructions, algorithms, or software for controlling the operation and performing the functions of respective device 212, 236, 239. In some embodiments, the one or more processors 303, 303', 303" may comprise a processor chipset including, for example and without limitation, one or more co-processors.

The memories 306, 306', 306" may be single memory devices or one or more memory devices at one or more memory locations that may include, without limitation, one or more of a random-access memory ("RAM"), a memory buffer, a hard drive, a database, an erasable programmable read only memory ("EPROM"), an electrically erasable programmable read only memory ("EEPROM"), a read only memory ("ROM"), a flash memory, hard disk, various layers of memory hierarchy, or any other non-transitory computer readable medium. The memories 306, 306', 306" may be on-chip or off-chip depending on the implementation of the one or more processors 303, 303', 303".

The memories 306, 306', 306" may be used to store any type of instructions 321, 321', 321" associated with algorithms, processes, or operations for controlling the general functions and operations of the devices 212, 236, 239. The instructions 321, 321', 321" may be any form of software, including, without limitation, firmware, executable applications, etc. Execution of the instructions 321, 321', 321" by the respective one or more processors 303, 303', 303" will impart the functionalities of the devices 212, 236, 239 associated with the presently disclosed technique as discussed below.

The communications interfaces 309, 309', 309" may permit the respective network device 212, 236, 239 to directly or indirectly (via, for example, a monitor mount) communicate with one or more computing networks and devices, workstations, consoles, computers, monitoring equipment, alert systems, and/or mobile devices (e.g., a mobile phone, tablet, or other hand-held display device). The communications interfaces 309, 309', 309" may include various network cards, network interfaces, communication channels, wireless radio modules, cloud, antennas, and/or circuitry to permit wired and wireless communications with such computing networks and devices.

The communications interfaces 309, 309', 309" may be used to implement, for example, a BLUETOOTH^{®} connection, a cellular network connection, and/or a WIFI^{®} connection with such computing networks and devices. Example wireless communication connections implemented using the communication interfaces 309, 309', 309" include wireless connections that operate in accordance with, but are not limited to, IEEE 802.11 protocol, a Radio Frequency For Consumer Electronics ("RF4CE") protocol, 5G cellular, and/or IEEE 802.15.4 protocol (e.g., ZigBee^{®} protocol). In essence, any wireless communication protocol may be used.

Additionally, the communications interfaces 309, 309', 309" may permit direct (i.e., device-to-device) communications (e.g., messaging, signal exchange, etc.) such as from, for example, a universal serial bus ("USB") connection or other communication protocol interface. The communications interfaces 309, 309', 309" may also permit direct device-to-device connection to other devices such as to a tablet, computer, or similar electronic device; or to an external storage device or memory.

Those skilled in the art will appreciate that the implementation of the sensor interface 312 will turn strongly on the physiological parameters being monitored. Different kinds of data collected by different kinds of sensors will typically be conditioned differently and, so, the implementation of the sensor interface 312 will differ. However, although not required, most implementations of the sensor interface 312 will include analog-to-digital conversion of the data. Furthermore, as discussed elsewhere, not all medical devices will be patient monitors nor will they necessarily interface with sensors. Some medical devices therefore may omit a sensor interface.

Note that the various components of devices 212, 236, 239 may be implemented differently in any given embodiment. As a non-limiting example, in one embodiment the one or more processors 303 may be a single microprocessor while the one or more processors 303' may be a processor chipset. Or, the memory 306 may be implemented in a single RAM device while the memory 306" may be implemented in a redundant array of independent disks. Those in the art having the benefit of this disclosure will appreciate still other examples of differences in implementation-specific differences across embodiments.

Returning to FIG. 2, the patient monitor 212 acquires data as described above and then processes and analyzes the data. As part of the processing and analyzing of the acquired data, the medical device 212 may detect an alarm condition. If so, an alarm is formulated and communicated. It is customary to announce the alarm using audio or visual cues. For example, the alarm may include, without limitation, a broadcast audio signal (e.g. a buzzing or beeping sound) and/or a visual signal (e.g., a flashing light). The alarm may also be communicated through the display 315. The alarm may also be transmitted to, for example, and without limitation, a centralized monitoring station (not shown), such as a nurses' station, over the computing system 224.

Note that some medical devices might acquire data and transmit it without processing and/or analyzing before transmission. In these situations, alarms may be generated for other reasons and in other ways. The presently disclosed technique admits wide variation in detection, transmission, and aggregation of the alarm data.

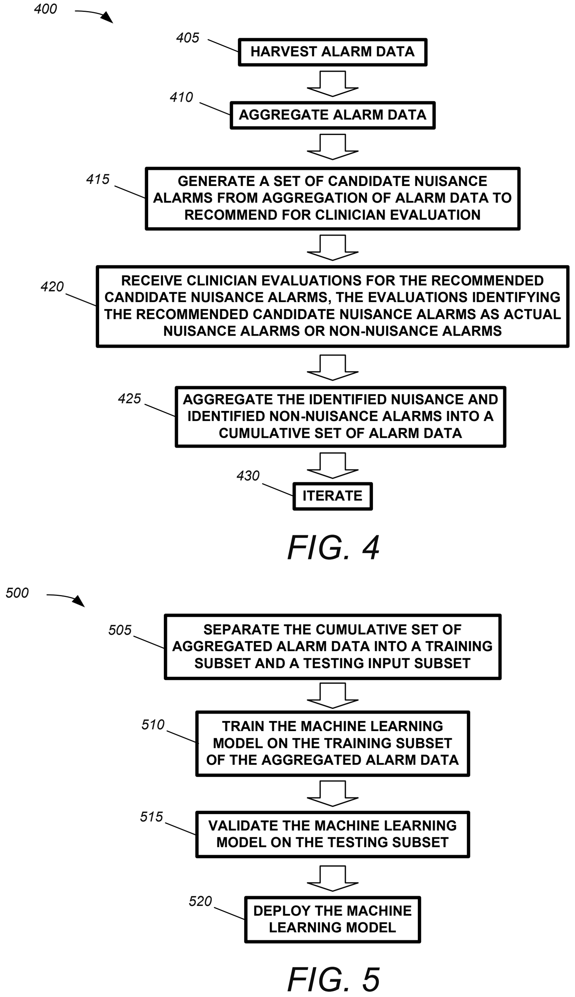

Accordingly, as shown in FIG. 4, the scenario 200 includes a method 400 in which the scenario begins with harvesting of alarms data (at 405) that are then harvested for aggregation (at 410) along with associated information-alarm data-as described above. The alarm data is harvested as the alarms occur and are, therefore, "organic" data-that is, the alarm data is not synthetic data generated solely for purposes of training or testing. The method 400 may be implemented using, for example, the workstation 236 in FIG. 2. More particularly, the method 400 may be implemented by the one or more processors 303" executing the instructions 321" shown in FIG. 3C.

As discussed above, the alarm data that is harvested may include information regarding the alarm itself, such as alarm type, priority, type of device issuing the alarm, date and time. The harvested alarm data may include relevant conditions that triggered the alarm, such as a parameter value for a limit alert or a battery level for a low battery alert. Other data that may be accumulated may include the time of day the alarm triggered, the time elapsed since the last alarm of the same type was announced, time elapsed in which the alarm was active, and correlation with other active alarms, for example.

Limit alerts can be set and adjusted for different physiological parameters, including for example heart rate (HR), respiration rate (RR), temperature (T), and/or SPO₂. For example, limit alerts can be adjusted using the machine learning algorithm and/or clinician feedback described herein to adjust limit alerts by an amount ranging from 0.1 - 0.5%, 0.1 - 2%, 0.5 - 5%, 0.2 - 4%, 2 - 5%, 5 - 10%, 10 - 25%, 5 - 15%, 25 - 35 % or 0.1 - 35%. Limit alerts can be set and adjusted for different physiological parameters according to alarm frequency, clinician feedback, alarm ranking, or through any combination of the above. Limit alerts can be set and adjusted by the machine learning algorithm for different physiological parameters according to alarm frequency, clinician feedback, alarm ranking, or through any combination of the above.

In some examples, individual parameter alarm limits may be adjusted using a set of data from the prior 48 hours, from the prior 24 hours, from the prior 12 hours, from the prior 6 hours, from the prior 3 hours, or from the prior 1 hour. In some examples, alarm limits are adjusted post-operatively for a given time frame to increase sensitivity. In some examples, the minimum number of successive abnormal reading needed for an alarm is adjusted (i.e. annunciation delay interval may be increased if minimum number of abnormal readings is increased). In some examples, alarm limits may be increased during the daytime and decreased during the nighttime, for example, for parameters including heartrate.

In some examples, the machine learning algorithm may be configured to turn off an alarming device if is not in use (no patient admitted, in standby, etc.). In some examples, the machine learning algorithm may be configured to silence or pause low-priority alarms for a brief period after caregiver acknowledgment and may be configured to continue alarm annunciation only if the condition worsens or persists.

In some examples, the machine learning algorithm may be configured to enable flood alarm protection and aggregate similar alarms within a short window and present a single summary notification. In some examples, the machine learning algorithm may be configured to temporarily suppress physiological alarms when a technical issue is present that can cause false positives. For example, if ECG leads off/poor contact is detected, the machine learning algorithm may be configured to disable arrhythmia alarms until the condition goes away. In another example, for SpO2 low alarms, the machine learning algorithm may be configured to disable alarms when signal quality is poor and technical alarms for "Check sensor/contact" until quality recovers. In another example, during NIBP inflation, the machine learning algorithm may be configured to temporarily suspend alarms to avoid inflation-related artifacts (SpO2) and reenable immediately after the cycle.

In some embodiments, the aggregated alarm data may also include contextual information for the alarm. For example, the location of the alarm may be aggregated. Other contextual information such as equipment configuration, equipment changes, configuration changes, maintenance, adjustments responsive to the alarm, and whether the alarm was flagged as a potential nuisance alarm. Some embodiments may even include images or biometric data captured from caregivers in the process of dealing with the alarm. For another example, a medical device may be equipped with a camera and/or a biometric sensor to capture the caregiver's countenance or body language and/or biometric data representative of the caregiver's physiological response to the alarm.

In some embodiments, the alarm data may include, for example, an indication of whether a caregiver silenced or otherwise responded to the alarm. (Note that, in some embodiments, the caregiver may also flag the alarm as a nuisance alarm when silencing the alarm. This determination may be included in the alarm data and used later in mitigating alarm fatigue as described herein.) For example, some medical devices that issue alarms permit a clinician or other caregiver to silence or mute an audio alarm, or to dismiss a visual alarm. These and other forms of clinician feedback at the bedside, or the point of origin for the alarm, at a monitoring station, or some location where a clinician is in a position to react may be harvested and aggregated.

The harvested alarm data is then aggregated (at 410) into a cumulative set of aggregated alarm data. The aggregation may occur in various ways depending on the implementation. For example, in FIG. 2, the medical device 212 may message the alarm data to the work space 227 over the computing system 224 using packet switched networking principles. Or the medical device may broadcast or multicast the alarm information 245. Either way, upon receiving the alarm data 245, the work space 227 may then aggregate the alarm data 245 by storing it on the server 239. Note that, in some embodiments, the receipt and storage attributed to the work space 227 may be performed by the workstation 236 using the server 239 or the server 239 directly.

Next, a set of candidate nuisance alarms to recommend for clinician evaluation is generated from the aggregated alarm data (at 415). The set is generated in the illustrated embodiments by applying a "classifier" (e.g., the classifier 330, shown in FIG. 3), which is a trained ML model as described above. If the classifier is not previously trained, then it may be trained.

The training of the ML model in the disclosed embodiments is supervised machine learning technique in which a labeled data set is used to train the ML model used to classify the alarms. In general terms, "training" is the term used to describe the process in which the ML model learns relationships between the input and output in order to determine the output, or classification, of the input. There are a number techniques that include, for example and without limitation, logistic regression, decision tree classifier, K nearest neighbor classifier, random forest classifier, and neural networks. Any suitable supervised training technique may be used.

FIG. 5 depicts a workflow 500 for training and deploying the ML model. The workflow 500 begins with separating (at 505) the cumulative set of aggregated alarm data (e.g., aggregated alarm data 245, FIG. 2) into a training subset (e.g., training subset 248, FIG. 2) and a testing subset (e.g., testing subset 251, FIG. 1). Note that the aggregated data should already be labeled prior to the splitting and the illustrated workflow 500 presumes that the data has already been labeled. Once the training subset has been labeled and separated from the testing subset, the machine learning model (e.g., the classifier 330, FIG. 3) is then trained (at 510) on the training subset of the aggregated alarm data. The trained ML model is then validated (at 515) on the testing subset. Once validated, the ML model is then deployed (at 520).

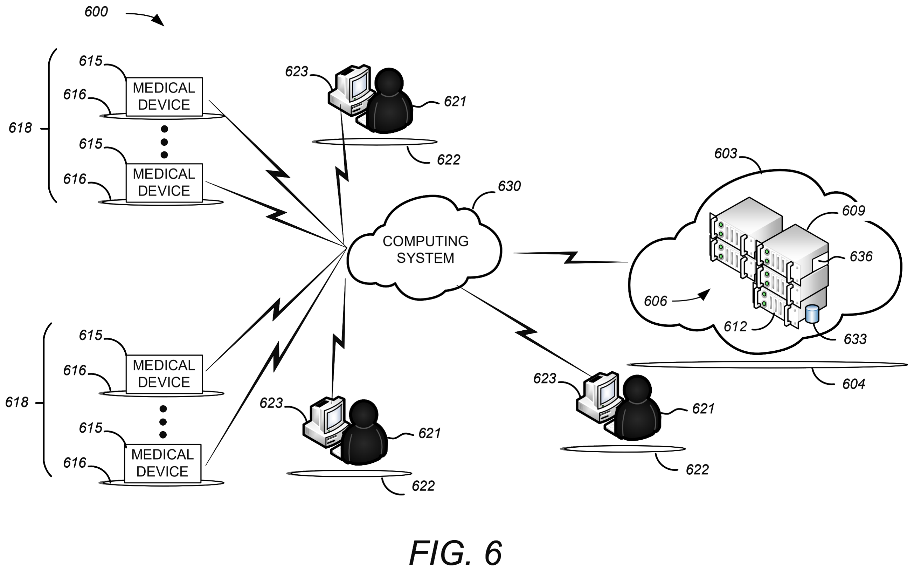

The deployment (at 520) of the trained ML model will depend upon the implementation of the technological system. In an implementation such as the one shown in FIG. 2, the deployment can include transmitting the trained machine learning model to a user location (e.g., location 233, FIG. 2). Or, in cloud-based implementations, deployment may comprise releasing the trained machine learning model in a cloud and permitting access thereto. One such embodiment is disclosed below relative to FIG. 6.

Receiving (at 420) clinician evaluations for the recommended candidate nuisance alarms, the evaluations identifying the recommended candidate nuisance alarms as actual nuisance alarms or non-nuisance alarms. In particular, the set of potential nuisance alarms may be displayed to, for example, the clinician 242 shown in FIG. 2 using the workstation 236. More particularly, the one or more processors 303" executing the instructions 321" shown in FIG. 3C may receive interact through the GUI 324" and the display 315". Depending on the implementation, the clinician may designate candidate nuisance alarms as either actual nuisance alarms or actual non-nuisance alarms. In some embodiments, the identity of non-nuisance alarms may be inferred from the lack of designation as actual nuisance alarms. In still other implementations, whether a candidate nuisance alarm is a non-nuisance alarm may be immaterial and, thus, such categorization may be omitted by neither designating nor inferring such status.

The method 400 then aggregates (at 425) the identified nuisance and identified non-nuisance alarms into a cumulative set of alarm data. The clinician's feedback identifying the actual nuisance and non-nuisance alarms as such then becomes information associated with the alarms aggregated as a part of the aggregated alarm data. Not all embodiments are so limited, however. In some embodiments the identification of the candidate nuisance alarms can be rolled back into the medical monitoring system in lieu of, or in addition to, aggregating those identifications into the cumulative set of alarm data.

For example, in the scenario 200 of FIG. 2, the patient monitor 212 is programmed to detect any number of alarm conditions and generate any number of alarms. This functionality arises from the execution of the one or more processors 303 executing the instructions 321, both shown in FIG. 3A. Once an alarm is identified as a nuisance alarm by the clinician feedback, a new configuration or setting for the instructions 321 can then be generated and transmitted to the patient monitor 212 over the computer system 224. The new configuration or setting may, for example, program the patient monitor 212 to mute an alarm. Or the new configuration or setting may adjust the parameters used in detecting the alarm that has been identified as a nuisance. Those skilled in the art will realize still other ways in which the identification of candidate alarms as actual nuisance alarms can be used to improve the functioning of the technological system.

The method 400 then iterates (at 430) the above steps. Note that in subsequent iterations, a different clinician may provide feedback such that multiple iterations can provide or reflect a broader consensus among clinicians as to what constitutes a nuisance alarm. Furthermore, as one iteration is being performed, the harvesting and aggregation of the alarm can continue so that the results of the process can involve a more comprehensive data set. Still further, multiple iterations may refine and emphasize what are truly nuisance alarms and what are non-nuisance alarms.

FIG. 6 depicts a scenario 600 in which the technological system 100 of FIG. 1 may be implemented according to one or more examples. A cloud computing system 600 at a location 604 includes a plurality of computing resources 606 that have been allocated to the implementation of the presently disclosed technique. The allocated resources may include, for example, processes resources 609 and memory or storage resources 612. The scenario 600 further includes a plurality of medical devices 615, each at a respective location 616 and the medical devices grouped in clusters 618. A plurality of clinicians 621, each at a respective location 622, also shown, each at a respective workstation 623. The locations 604, 616, 622 may be local or remote as discussed above. The computing devices (e.g., medical devices 615, workstations 623, cloud computing resources 606) communicate with one another over the computing system 630, also as discussed above.

Theoretically, there is no limitation on the number of medical devices 615, workstations 623 or locations 616 subject only to the adequate availability of other computing resources. Such "other" computing resources may include, for example, bandwidth across the computing system 630 and computing resources 606 in the cloud computing system 603. Accordingly, the number of medical devices 615, workstations 623, and computing resources 606 are representative only and alternative embodiments may include more or fewer computing devices. Similarly, the number of locations 616, 622, and 604 may vary by implementation. Although only one medical device 615 is shown per location 616, each location 616 may host many medical devices 615. Similarly, any number of workstations 623 may be located in any given location 622 and the cloud computing resources can be distributed across multiple locations 604.

The medical devices 615 operate in accordance with their programs to execute their programmed functionality relative to the patients (not shown in FIG. 6) to which they are assigned. In the course of these operations, alarms are detected and addressed by bedside caregivers or clinicians, neither of which are shown. In some cases, the clinicians 621 may be monitoring the medical devices 615 via the workstations 623 and address the alarms. How an alarm may be addressed will depend on the condition that triggered the alarm. Addressing the alarm may be as complicated as delivering emergency treatment or as simple as muting the alarm.

Upon the occurrence of an alarm, the medical device 615 transmits the alarm data as discussed above generated by the occurrence of the alarm. The alarm data may also include, in some embodiments, clinician feedback as to whether the alarm is a nuisance alarm. For example, if the clinician mutes the alarms quickly by addressing the underlying cause, it may be inferred that the alarm is a nuisance alarm. That information can then be included in the transmitted alarm data.

All such alarm data is transmitted over the computing system 630 to the allocated computing resources 606 of the cloud computing system 603. The alarm data received over the computing system 630 from all the medical devices 615 is aggregated in the allocated storage resources 612. The aggregated alarm data 633 is then operated on by the allocated processing resources 609, including both hardware and software resources 636, as described above relative to FIG. 1. The hardware and software resources 636 classify the alarms as nuisance or non-nuisance, prioritizes them, presents them to at least one clinician 621. The hardware and software resources 636 then receive the feedback of at least one of the clinicians 621. The resultant set of actual nuisance alarms can then be used in one of several ways as described herein to help mitigate alarm fatigue.

One aspect of the presently disclosed embodiment is the direct incorporation of clinician feedback in the identification of nuisance alarms. Clinician feedback is directly incorporated (e.g., at 420, FIG. 4) when the clinician identifies candidate nuisance alarms as actual nuisance alarms or non-nuisance alarms. In some embodiments, clinician feedback is also directly incorporated through the harvesting of alarm data that indicates, for example, whether a clinician has muted or dismissed the alarm and how quickly they did so. Clinician input incorporated in this second manner then also is acted upon in the generation (e.g., at 415, FIG. 4) of the set of candidate nuisance alarms from the aggregated alarm data.

The embodiments disclosed above incorporate the clinicians' feedback based on the aggregated alarm data into the medical monitoring system from which the alarm data is harvested. However, the presently disclosed technique is not so limited and may be incorporated into other medical monitoring systems. While this approach will also yield desirable results in reducing nuisance alarms and, hence, alarm fatigue, better it is expected that better results will be obtained when incorporating the clinicians' feedback into the medical monitoring system from which it is harvested. In embodiments in which the clinician's feedback is incorporated into a medical monitoring system other than that from which it is harvested, quality of results will be proportional to the similarity between the two systems in terms of composition and scale.

The discussion above presents a number of acts in a certain order in accordance with a particular chronology. This is illustrative only and the order and chronology are not necessarily required or implemented in all embodiments. The method descriptions and the process flow diagrams set forth herein are provided merely as illustrative examples and are not intended to require or imply that the operations of the various embodiments must be performed in the order presented.

As will be appreciated by one of skill in the art the order of operations in the foregoing embodiments may be performed in any order. Words such as "thereafter," "then," "next," etc. are not intended to limit the order of the operations; these words are simply used to guide the reader through the description of the methods. Exceptions may be found where the language describing the acts imposes such order. However, the mere order of presentation does not necessarily impose that order on the claimed subject matter.

For example, returning to FIG. 4, the steps of the method 400 are shown in a certain order. Receiving clinician evaluations (at 420) waits for the generation (at 415) of the set of recommended candidate nuisance alarms. However, harvesting (at 405) and aggregation (at 410) of alarm data can continue to occur even while the set of recommended candidate nuisance alarms is generated (at 415) and the clinician gives the feedback in the form of the evaluations (at 420). Those in the art having the benefit of this disclosure will realize still other situations in which this principle may be realized.

Note that the alarm data discussed above is "organic" or "actual" alarm data, as opposed to "synthetic". That is, the aggregated alarm data is harvested from alarms that actually occur in a medical monitoring system as opposed synthetic data generated for testing purposes. Thus, as the clinicians' recommendations are incorporated to mute or otherwise manage nuisance alarms, the result is a healthcare environment with objectively fewer nuisance alarms leading the lesser alarm fatigue. This is particularly true when the clinicians' feedback is incorporated back into the medical monitoring system from which it was harvested.

The presently disclosed technique therefore improves the efficiency and operation of a technological system that may be used in managing alarm fatigue. The technique improves the operation of the technological system by reducing false alarms. It also has a practical application in helping to reduce the impact of alarm fatigue that might result from use of the technological system. This solution's workflow also aids with the problem of annotating data for the ML model. The collected data collected used for training the ML must typically be annotated with the correct labels, and this can be a labor-intensive job in conventional practice. This labor can be mitigated by having the hospital involved in the labeling as part of the workflow for reviewing the results.

The expressions such as "include" and "may include" which may be used in the present disclosure denote the presence of the disclosed functions, operations, and constituent elements, and do not limit the presence of one or more additional functions, operations, and constituent elements. In the present disclosure, terms such as "include" and/or "have", may be construed to denote a certain characteristic, number, operation, constituent element, component or a combination thereof, but should not be construed to exclude the existence of or a possibility of the addition of one or more other characteristics, numbers, operations, constituent elements, components or combinations thereof.

As used herein, the article "a" is intended to have its ordinary meaning in the patent arts, namely "one or more." Herein, the term "about" when applied to a value generally means within the tolerance range of the equipment used to produce the value, or in some examples, means plus or minus 10%, or plus or minus 5%, or plus or minus 1%, unless otherwise expressly specified. Further, herein the term "substantially" as used herein means a majority, or almost all, or all, or an amount with a range of about 51% to about 100%, for example. Moreover, examples herein are intended to be illustrative only and are presented for discussion purposes and not by way of limitation.

As used herein, to "provide" an item means to have possession of and/or control over the item. This may include, for example, forming (or assembling) some or all of the item from its constituent materials and/or, obtaining possession of and/or control over an already-formed item.

Unless otherwise defined, all terms including technical and/or scientific terms used herein have the same meaning as commonly understood by one of ordinary skill in the art to which the present disclosure pertains. In addition, unless otherwise defined, all terms defined in generally used dictionaries may not be overly interpreted. In the preceding, details are set forth to provide a more thorough explanation of the embodiments. However, it will be apparent to those skilled in the art that embodiments may be practiced without these specific details. In other instances, well-known structures and devices are shown in block diagram form or in a schematic view rather than in detail in order to avoid obscuring the embodiments. In addition, features of the different embodiments described hereinafter may be combined with each other, unless specifically noted otherwise. For example, variations or modifications described with respect to one of the embodiments may also be applicable to other embodiments unless noted to the contrary.

Further, equivalent or like elements or elements with equivalent or like functionality are denoted in the preceding description with equivalent or like reference numerals. As the same or functionally equivalent elements are given the same reference numbers in the figures, a repeated description for elements provided with the same reference numbers may be omitted. Hence, descriptions provided for elements having the same or like reference numbers are mutually exchangeable.

It will be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present. Other words used to describe the relationship between elements should be interpreted in a like fashion (*e.g.*, "between" versus "directly between," "adjacent" versus "directly adjacent," *etc.*).

In the present disclosure, expressions including ordinal numbers, such as "first", "second", and/or the like, may modify various elements. However, such elements are not limited by the above expressions. For example, the above expressions do not limit the sequence and/or importance of the elements. The above expressions are used merely for the purpose of distinguishing an element from the other elements. For example, a first box and a second box indicate different boxes, although both are boxes. For further example, a first element could be termed a second element, and similarly, a second element could also be termed a first element without departing from the scope of the present disclosure.

A sensor refers to a component which converts a physical quantity to be measured to an electric signal, for example, a current signal or a voltage signal. The physical quantity may for example comprise electromagnetic radiation (e.g., photons of infrared or visible light), a magnetic field, an electric field, a pressure, a force, a temperature, a current, or a voltage, but is not limited thereto.

Use of the phrases "capable of," "capable to," "operable to," "configured to," or "programmed to" in one or more embodiments, refers to some apparatus, logic, hardware, and/or element designed in such a way to enable the use of the apparatus, logic, hardware, and/or element in a specified manner. Use of the phrase "exceed" in one or more embodiments, indicates that a measured value could be higher than a pre-determined threshold (e.g., an upper threshold), or lower than a pre-determined threshold (e.g., a lower threshold). When a pre-determined threshold range (defined by an upper threshold and a lower threshold) is used, the use of the phrase "exceed" in one or more embodiments could also indicate a measured value is outside the pre-determined threshold range (e.g., higher than the upper threshold or lower than the lower threshold). The subject matter of the present disclosure is provided as examples of apparatus, systems, methods, circuits, and programs for performing the features described in the present disclosure. However, further features or variations are contemplated in addition to the features described above. It is contemplated that the implementation of the components and functions of the present disclosure can be done with any newly arising technology that may replace any of the above-implemented technologies.

Various modifications to the disclosure will be readily apparent to those skilled in the art, and the generic principles defined herein may be applied to other variations without departing from the scope of the present disclosure. Throughout the present disclosure the terms "example," "examples," or "exemplary" indicate examples or instances and do not imply or require any preference for the noted examples. Thus, the present disclosure is not to be limited to the examples and designs described herein but is to be accorded the widest scope consistent with the principles and novel features disclosed.

This concludes the detailed description. The particular embodiments disclosed above are illustrative only, as the invention may be modified and practiced in different but equivalent manners apparent to those skilled in the art having the benefit of the teachings herein. Furthermore, no limitations are intended to the details of construction or design herein shown, other than as described in the claims below. It is therefore evident that the particular embodiments disclosed above may be altered or modified and all such variations are considered within the scope of the invention as defined by the appended claims.

The present disclosure includes the subject matter set out in the following numbered Clauses:
Clause 1. A computer-implemented method for managing alarm fatigue, comprising:
   generating a set of candidate nuisance alarms from an aggregation of alarm data to recommend for clinician evaluation;
   receiving clinician evaluations for the recommended candidate nuisance alarms, the evaluations identifying the recommended candidate nuisance alarms as actual nuisance alarms or non-nuisance alarms;
   aggregating the identified nuisance and identified non-nuisance alarms into a cumulative set of alarm data; and
   iterating the above steps.
Clause 2. The computer-implemented method of Clause 1, further comprising:
   harvesting alarm data as alarms occur; and
   aggregating the harvested alarm data into a cumulative set of aggregated alarm data from which the set of candidate nuisance alarms is generated.
Clause 3. The computer-implemented method of Clause 2, wherein harvesting the alarm data includes harvesting clinician feedback as to whether one or more of the alarms is a nuisance alarm.
Clause 4. The computer-implemented method of any one of Clauses 2 or 3, wherein the aggregated alarm data includes clinician feedback as to whether one or more of the alarms is a nuisance alarm.
Clause 5. The computer-implemented method of any one of Clauses 2 to 4, further comprising training a machine learning model, the training including:
   separating the cumulative set of aggregated alarm data into a training subset and a testing subset;
   training the machine learning model on the training subset of the aggregated alarm data;
   validating the machine learning model on the testing subset; and
   deploying the machine learning model.
Clause 6. The computer-implemented method of any one of Clauses 1 to 5, further comprising:
   training a machine learning model on a training subset of aggregated alarm data to classify a first plurality of alarms as nuisance alarms or non-nuisance alarms; and
   deploying the trained machine learning model.
Clause 7. The computer-implemented method of Clause 6, wherein deploying the trained machine learning model includes:
   releasing the trained machine learning model in a cloud and permitting access thereto; or
   transmitting the trained machine learning model to a user location.
Clause 8. The computer-implemented method of any one of Clauses 1 to 7, further comprising:
   generating from the classified non-nuisance alarms a set of candidate non-nuisance alarms to recommend for clinician evaluation; and
   receiving clinician evaluations for the recommended candidate non-nuisance alarms, the evaluations identifying the recommended candidate non-nuisance alarms as actual nuisance alarms or non-nuisance alarms.
Clause 9. The computer-implemented method of any one of Clauses 1 to 8, further comprising presenting the set of candidate nuisance alarms for clinician evaluation.
Clause 10. The computer-implemented method of any one of Clauses 1 to 9, wherein the set of candidate nuisance alarms is a prioritized set.
Clause 11. The computer-implemented method of Clause 8, wherein receiving clinician evaluations includes receiving a clinician input through a user interface as to whether each of the candidate nuisance alarms on the presented set is a nuisance alarm or a non-nuisance alarm.
Clause 12. The computer-implemented method of Clause 11, wherein receiving clinician evaluations includes receiving a clinician input as to which candidate nuisance alarms are non-nuisance alarms is inferred from the clinician input as to which candidate nuisance alarms is a non-nuisance alarm.
Clause 13. The computer-implemented method of any one of Clauses 1 to 12,
   further comprising harvesting alarm data as alarms occur, the harvesting including image or biometric data acquisition of a clinician as the clinician encounters an alarm; and
   wherein receiving clinician evaluations includes analyzing the acquired image or biometric data.
Clause 14. The computer-implemented method of any one of Clauses 1 to 13, wherein generating the set of candidate nuisance alarms includes applying a machine learning model trained on a training subset of aggregated alarm data to an input subset of the aggregated data.
Clause 15. The computer-implemented method of Clause 14, wherein aggregating the identified nuisance and identified non-nuisance alarms into a cumulative set of alarm data includes aggregating the identified nuisance alarms and identified non-nuisance alarms into the training subset, the training subset, or an input subset, or a combination thereof to create or supplement the cumulative data set.
Clause 16. The computer-implemented method of any one of Clauses 1 to 15,
   wherein aggregating the identified nuisance and identified non-nuisance alarms into a cumulative set of alarm data; and
   the method further comprises separating the cumulative set of alarm data into a training subset and a training subset.
Clause 17. The computer-implemented method of any one of Clauses 1 to 16, wherein aggregated alarm data includes clinician feedback as to whether one or more of the alarms is a nuisance alarm.
Clause 18. A method for use in reducing alarm fatigue, the method comprising:
   separating a set of aggregated alarm data into a training subset and a testing subset;
   generating a set of candidate nuisance alarms by applying a machine learning model trained on the training subset to the testing subset to classify a plurality of alarms as nuisance alarms or non-nuisance alarms; and
   prioritizing the set of candidate nuisance alarms.
Clause 19. A method for use in reducing alarm fatigue, the method comprising:
   presenting a prioritized set of candidate nuisance alarms for clinician evaluation, the set generated from identifying the candidate nuisance alarms in a set of aggregated alarm data;
   receiving clinician evaluations for the recommended candidate nuisance alarms, the evaluations identifying the recommended candidate nuisance alarms as actual nuisance alarms or non-nuisance alarms; and
   aggregating the identified nuisance and identified non-nuisance alarms into the set of aggregated alarm data.
Clause 20. A computing system, comprising:
   one or more processors; and
   a memory on which resides,
      an aggregation of alarm data; and
      a set of instructions that, when executed by the one or more processors, performs a method for managing alarm fatigue, the method comprising:
         generating a set of candidate nuisance alarms from the aggregation of alarm data to recommend for clinician evaluation;
         receiving clinician evaluations for the recommended candidate nuisance alarms, the evaluations identifying the recommended candidate nuisance alarms as actual nuisance alarms or non-nuisance alarms;
         aggregating the identified nuisance and identified non-nuisance alarms into a cumulative set of alarm data; and
         iterating the above steps.
Clause 21. The computing system of Clause 20, wherein the at least one of the one or more processors and the memory include allocated cloud computing system resources.
Clause 22. The computing system of Clause 20 or 21, wherein the one or more processors and the memory comprise a part of a single computing device.
Clause 23. The computing system of any one of Clauses 20 to 22, wherein the one or more processors and the memory are distributed across multiple computing devices.
Clause 24. A technological system, comprising:
   a plurality of medical devices programmed to:
      detect alarm conditions;
      announce alarms responsive to detecting alarm conditions; and
      transmit alarm data associated with the alarm; and
   a computing apparatus, comprising:
      one or more processors; and
      a memory on which resides,
         an aggregation of alarm data; and
         a set of instructions that, when executed by the one or more processors, performs a method for managing alarm fatigue, the method comprising:
            generating a set of candidate nuisance alarms from the aggregation of alarm data to recommend for clinician evaluation;
            receiving clinician evaluations for the recommended candidate nuisance alarms, the evaluations identifying the recommended candidate nuisance alarms as actual nuisance alarms or non-nuisance alarms;
            aggregating the identified nuisance and identified non-nuisance alarms into a cumulative set of alarm data; and
            iterating the above steps.
Clause 25. A computer-implemented method for managing alarm fatigue in a medical monitoring environment, comprising:
   identifying nuisance alarms occurring in the medical monitoring environment, including:
      harvesting alarm data from a plurality of medical devices;
      aggregating the alarm data;
      applying a trained machine learning model to the aggregated alarm data to generate a prioritized set of potential nuisance alarms; and
      receiving clinician feedback identifying actual nuisance alarms from among the potential nuisance alarms; and
   updating the programming of the plurality of medical devices to reflect the newly identified actual nuisance alarms.
Clause 26. A system implementing the method of Clause 25.

## Claims

1. A computer-implemented method for managing alarm fatigue, comprising:
generating a set of candidate nuisance alarms from an aggregation of alarm data to recommend for clinician evaluation;
receiving clinician evaluations for the recommended candidate nuisance alarms, the evaluations identifying the recommended candidate nuisance alarms as actual nuisance alarms or non-nuisance alarms;
aggregating the identified nuisance and identified non-nuisance alarms into a cumulative set of alarm data; and
iterating the above steps.

2. The computer-implemented method of claim 1,
further comprising, in each iteration after receiving the aggregated alarm data:
classifying the alarms whose data has been aggregated as potential nuisance alarms or non-nuisance alarms; and
prioritizing the classified alarms to generate a prioritized set of potential nuisance alarms and recommendations for corrective actions,
wherein the potential nuisance alarms comprise the recommended candidate nuisance alarms.

3. The computer-implemented method of claim 2, wherein the potential nuisance alarms include a low battery technical alarm, a parameter high limit, a parameter low limit, or a hardware failure, or combinations thereof.

4. The computer-implemented method of claim 2 or 3, further comprising presenting one or more recommended actions for a potential nuisance alarm, the recommended actions including adjusting the limit value of a parameter.

5. The computer-implemented method of any one of claims 1 to 4, wherein the candidate alarms are prioritized by one or more factors including one or more of frequency and confidence level that the candidate alarm is a nuisance alarm, alarm severity, alarm location, alarm type, or severity of potential impact on the patient's physical condition.

6. A computing system, comprising:
one or more processors; and
a memory on which resides,
an aggregation of alarm data; and
a set of instructions that, when executed by the one or more processors, performs a method for managing alarm fatigue, the method comprising:
generating a set of candidate nuisance alarms from the aggregation of alarm data to recommend for clinician evaluation;
receiving clinician evaluations for the recommended candidate nuisance alarms, the evaluations identifying the recommended candidate nuisance alarms as actual nuisance alarms or non-nuisance alarms;
aggregating the identified nuisance and identified non-nuisance alarms into a cumulative set of alarm data; and
iterating the above steps.

7. The computing system of claim 6, wherein the programmed method further comprises:
in each iteration after receiving the aggregated alarm data:
classifying the alarms whose data has been aggregated as potential nuisance alarms or non-nuisance alarms; and
prioritizing the classified alarms to generate a prioritized set of potential nuisance alarms and recommendations for corrective actions,
wherein the potential nuisance alarms comprise the recommended candidate nuisance alarms.

8. The computing system of claim 7, wherein the potential nuisance alarms include a low battery technical alarm, a parameter high limit, a parameter low limit, or an oxygen saturation (SpO2) hardware failure, or combinations thereof.

9. The computer system of claim 7 or 8, further comprising presenting one or more recommended actions for a potential nuisance alarm, the recommended actions including adjusting the limit value of a parameter.

10. The computing system of any one of claims 6 to 9, wherein the candidate alarms are prioritized.

11. The computing system of claim 10, wherein the candidate alarms are prioritized by one or more factors including one or more of frequency and confidence level that the candidate alarm is a nuisance alarm, alarm severity, alarm location, alarm type, or severity of potential impact on the patient's physical condition.

12. A technological system, comprising:
a plurality of medical devices programmed to:
detect alarm conditions;
announce alarms responsive to detecting alarm conditions; and
transmit alarm data associated with the alarm; and
a computing apparatus, comprising:
one or more processors; and
a memory on which resides,
an aggregation of alarm data; and
a set of instructions that, when executed by the one or more processors, performs a method for managing alarm fatigue, the method comprising:
generating a set of candidate nuisance alarms from the aggregation of alarm data to recommend for clinician evaluation;
receiving clinician evaluations for the recommended candidate nuisance alarms, the evaluations identifying the recommended candidate nuisance alarms as actual nuisance alarms or non-nuisance alarms;
aggregating the identified nuisance and identified non-nuisance alarms into a cumulative set of alarm data; and
iterating the above steps.

13. The technological system of claim 12, wherein the programmed method further comprises:
in each iteration after receiving the aggregated alarm data:
classifying the alarms whose data has been aggregated as potential nuisance alarms or non-nuisance alarms; and
prioritizing the classified alarms to generate a prioritized set of potential nuisance alarms and recommendations for corrective actions,
wherein the potential nuisance alarms comprise the recommended candidate nuisance alarms.

14. The technological system of claim 13, further comprising presenting one or more recommended actions for a potential nuisance alarm, the recommended actions including adjusting the limit value of a parameter.

15. The technological system of any one of claims 12 to 14, wherein the candidate alarms are prioritized by one or more factors including one or more of frequency and confidence level that the candidate alarm is a nuisance alarm, alarm severity, alarm location, alarm type, or severity of potential impact on the patient's physical condition.
